# EUROPEAN PATENT APPLICATION

(11) **EP 4 670 690 A1**
(43) Date of publication of application: **31.12.2025**
(21) Application number: 24185483.5
(22) Date of filing: 28.06.2024
(51) Int. Cl.: A61F 7/10, B33Y 80/00, A61N 5/00

(54) **WATER BOLUS WITH WATER VOLUME FLOW CONTROL**

(71) Applicant: Erasmus University Rotterdam Medical Center, 3015 GE Rotterdam (NL); Technische Universiteit Delft, 2628 CN Delft (NL); Beljaars, Marie Louise Martine, 3290 Schaffen-Diest (BE)
(72) Inventor: BELJAARS, Marie Louise Martine, 3290 Schaffen-Diest (BE); SÜMSER, Kemal, 3015 GE Rotterdam (NL); PAULIDES, Margarethus Marius, 3015 GE Rotterdam (NL); JANSEN, Kaspar Maria Bonaventura, 2628 CN Delft (NL); VAN RHOON, Gerard Cornelis, 3015 GE Rotterdam (NL)
(74) Representative: V.O.

(57) **Abstract**

Water bolus (200) for covering part of an outer body surface of a body part, the water bolus comprising:
- a flexible cover (202) for forming at least part of a container for containing a liquid; and
- an insert (100) provided inside the flexible cover,
wherein the insert is formed by interconnected struts (102) which form an open structure through which a liquid can flow.

## Description

### TECHNICAL FIELD

The aspects of the present disclosure relate to a water bolus for covering part of an outer body surface of a body part, in particular for use in hyperthermia treatment.

### BACKGROUND

Hyperthermia treatment is a method used for treating tumour cells by exposing body tissue of cancer patients to temperatures in the range of 40-44 °C. Hyperthermia stimulates blood perfusion leading, e.g., to a more effective delivery of drugs in tissues and the presence of more oxygen and, hence, the creation of more oxygen-radicals by ionizing radiation. Hyperthermia also sensitizes tissues to these DNA damaging agents in multiple ways, e.g. through blocking DNA damage repair. Healthy tissue can remain undamaged during hyperthermia treatment up to a temperature of 44 °C for up to an hour. The high temperature that is required for hyperthermia treatment is induced by exposing the patient's body tissue to electromagnetic waves.

While the tumour cells are being heated, the electromagnetic waves may cause skin burns on a patient's skin. Furthermore, electromagnetic waves may be hard to guide to the correct place inside the patient's body. To tackle these issues, a layer with water, a water bolus, is placed between electromagnetic wave generators and the skin of the patient as a cooling agent to prevent burns from the radiated heat and as a transferring agent for the electromagnetic energy. The electromagnetic waves typically have a frequency between 500 kHz and 2 GHz.

Known water boluses are formed as balloons through which water may be circulated. One side of the water bolus is arranged for skin contact, and preferably the contact between water bolus and skin is as good as possible meaning that no air gaps between the water bolus and the skin are desired. The fully water-filled water bolus pressed by the EM-applicator to the skin surface, however, does not provide a reliable skin contact, as for example folds or sharp wedges occur at the patient side of the water bolus which creates undesired air gaps between the patient's skin and the water bolus. At the site of the wedges the shape change may induce high concentration of the electromagnetic field resulting in local high heating and a toxicity risk to the patient. Next to the high heating, the wedges result in a poorly cooled region which in combination with the heating poses a high risk for burns.

Furthermore, the fully water-filled water bolus presses onto the body part of the patient that is being treated due to the weight and pressure of the water inside the water bolus. This pressing may be experienced as uncomfortable, for example when the body part is the patient's head, face or neck.

EP2744564B1 discloses the use of a foam insert, provided inside the water bolus. This foam insert increases the shape stability and decreases the amount of undesired folds at the patient side of the water bolus. Any insert has the disadvantage that it decreases the volume percentage of water in the water bolus which may decrease the volume average dielectric value and hence the guidance of the electromagnetic or acoustic energy.

Open-cell polyether foam is therefore used since it provides a low filling percentage, and hence allows most of the available volume inside the water bolus to be filled with water. The open cell structure of the foam further allows water to flow easily through the insert and thus through the water bolus which is beneficial for effectively cooling the patient's skin and obtaining a more uniform water temperature within the water bolus.

EP3669937A1 suggests using an insert formed by a plurality of linked, thin-walled, elongated, parallel, hollow structures.

### SUMMARY

The insert of EP3669937A1 already provides for additional stiffness and thus resistance against weight and pressure of water inside a water bolus. Furthermore, the insert is not too resistant against the shape of the patient, and thus allows for the water bolus to comply to the shape of the patient. It however remains an object to further improve an insert for a water bolus, in particular for use in hyperthermia treatment. Improvements may be found in manufacturability of the insert and/or bolus, shape stability of the insert and/or bolus, controllability of a flow of water through the insert, patient comfort, and/or in any other feature of the insert and/or bolus. With an increased controllability of the flow of water through the insert, for example, more or less cooling may be achieved in different regions of the water bolus. It has been observed that for example less cooling may be desired for areas of the body to be cooled with high blood flow through said area, and/or that more cooling may be desired for example at an area of the body to be cooled with scar tissue. Less cooling may for example be achieved by locally decreasing a flow speed of liquid through the water bolus, and/or by positioning a particular region of the water bolus further downstream in a flow path of liquid through the water bolus - considering that the temperature of the liquid flowing through the water bolus is gradually increased.

A first aspect of the present disclosure provides a water bolus for covering part of an outer body surface of a body part, the water bolus comprising a flexible cover for forming at least part of a container for containing a liquid, and an insert provided inside the flexible cover. The insert is formed by interconnected struts which form an open structure through which a liquid can flow.

When the insert is formed by interconnected struts which form an open structure through which a liquid can flow, the insert can be used to provide shape stability to the water bolus. Because the insert forms an open structure, a liquid, such as water, can flow through the insert. Furthermore, an open structure allows less volume inside the water bolus being occupied with the insert. Without the insert, the flexible cover when filled with liquid would be prone to collapse and/or to form sharp folds. A strut may also be referred to as a rod or a bar.

In the context of the present disclosure, a strut is defined as an elongated element. Typically, for any strut, the length of the strut exceeds a diameter or width of the strut. For example, the length of the strut may be at least 2 times, at least 4 times, or even at least 10 times the diameter or width of the strut. Struts of the same insert may generally have a similar length, or mutually different lengths. Any strut may have a generally cylindrical shape, but may also have any non-circular cross-sectional shape. Any strut may have an essentially constant cross-sectional shape, or may have a varying cross-sectional shape over the length of the strut. An insert may comprise any number of struts, for example over 50 struts, over 100 struts, over 1000 struts or even over 5000 struts.

An increase in the number of struts may allow for a more complex outer shape of the insert, which outer shape may be in general be adapted to follow an outer counter of any part of a human or animal body, in particular a neck and/or head of a human body. Additionally or alternatively, the arrangement of struts within the insert may become more complex with an increased number of struts. For example, an increase in the number of struts may allow for a local increase in strut density - also referred to as fill factor - within one or more regions of the insert, wherein an increase in strut density may for example be used to decrease cooling in a certain region. Further additionally or alternatively, an increase in the number of struts may allow for the flow path of liquid through the insert to become more complex, which in turn may result in increased control on what areas of a body against which a water bolus is positioned are cooled less or more. Even further additionally or alternatively, an increase in the number of struts may allow for more accurate control of the electromagnetic behaviour of the insert.

For any insert disclosed herein, a diameter or width of any strut may be between 0.2 and 2.0 mm, in particular between 0.5 and 1.0 mm. For any insert, and any strut, a length of the strut may be between 0.2 and 50 mm, in particular between 1 mm and 30 mm, further in particular between 5 and 15 mm. For any insert, the lengths of the majority or even all struts may be between 0.2 and 50 mm, in particular between 1 mm and 30 mm, further in particular between 5 and 15 mm. Within an insert, struts with different lengths may be provided. For example, a length of one or more struts in a first region of the insert may be shorter than a length of one or more struts in a second region of the insert, which second region is located adjacent to the first region. Locally changing a length, diameter, and/or width of struts may allow stiffness against compression of the insert to be locally changed and thus tuned. Any region, such as the first, second, third, fourth, fifth, and sixth region, discussed herein may be any arbitrary region at any arbitrary location within the insert, with any size and shape.

Similarly, a cross-sectional area of struts may be different for different struts of an insert. For example, a cross-sectional area of one or more struts in a third region of the insert may be different from a cross-sectional area of one or more struts in a fourth region of the insert, which fourth region is located adjacent to the third region. The first, second, third and fourth regions may be any arbitrary part of the insert. Locally changing a cross-sectional area of struts may allow stiffness against compression of the insert to be locally changed and thus tuned.

Any strut is preferably sufficiently flexible to allow buckling of the strut when using the insert as part of a water bolus. Buckling may occur when a body part of a patient is pressed against the insert, and the buckling allows the shape of the insert to deform - and thus to better comply with the shape of the body part of the patient.

When liquid can flow through the insert, a more homogenous liquid temperature may be achieved in the water bolus. Preferably, the total volume occupied by the insert is at most 30%, at most 20%, at most 10%, or even at most 5% of the volume occupied by liquid, in particular water, in the water bolus. Locally, in one or more regions of the insert, a fill factor of the insert may be between above 30%, above 50%, or even above 60%.

Typically, for any insert, a majority or even all of the struts are at both ends connected to at least two further struts. Interconnected struts can for any insert form polyhedrons, for example tetrahedrons, cubes, quadrilaterals, octahedrons, any other polyhedron, and any combination thereof.

The flexible cover may be formed by any flexible material, and typically is more flexible than the insert. The stiffness against compression of the flexible cover without the insert can be too low to support the weight of the flexible cover itself. Preferably, the shape of the water bolus is determined by the insert, rather than by the flexible cover. The flexible cover in use functions to contain a volume of liquid, in particular water, and to contain the insert. For any water bolus, the flexible cover may be formed by a film, for example formed from polypropylene (PP) or styrene ethylene butadiene styrene (SEBS), or from PE, PVC, PET, nylon, silicone, or PU. It is possible that the flexible cover is integrally formed with the insert, and as such the material of the insert preferably is suitable to merge with the material of the flexible cover.

As an option for any insert, a majority of the struts are at both ends connected to at least two further struts. Additionally, or alternatively, at least three struts of the insert of which outer ends are connected form a triangle. Further additionally, or alternatively, at least four struts of the insert of which outer ends are connected form a quadrilateral. Even further, additionally, or alternatively, at least four struts are connected at their respective outer ends and/or wherein at least five, six, or even more struts are connected at their respective outer ends. Any of these options may be combined with one or more of the other options. More in general, inserts are envisioned with any combination of any number of optional features disclosed herein.

Preferably, but not necessarily, for at least 95%, at least 99%, or even all struts of any insert disclosed herein, both outer ends of the strut are connected to an outer end of a further strut. When both outers ends of a strut are connected to an outer end of a further strut, it may be prevented than an outer end accidentally penetrates the flexible cover. Additionally or alternatively, a strut with an outer end which is not connected to a further strut may drastically decrease local stiffness of the insert. More in general, for any insert, it is preferred that the insert is essentially free of sharp and/or protruding elements which might puncture the flexible cover.

When the insert is connected to an inside of the flexible cover, the insert may further improve shape stability of the water bolus. For example, the insert may be glued, otherwise adhered, welded, or otherwise connected to the inside of the flexible cover. As a particular option. a plurality of the struts of the insert, and/or any other part of the insert, are connected to an inside of the flexible cover, for example by an adhesive. Additionally or alternatively, when the insert is connected to an inside of the flexible cover, it may be prevented that water flows around the insert instead of as preferred through the insert.

Any insert disclosed herein may be formed using additive manufacturing - otherwise referred to as 3D-printing. In particular, fused deposition modelling (FDM) may be used to form the insert. Alternatively, Selective Laser Sintering (SLS), Multi Jet Fusion (MJF), or any other additive manufacturing method may be used to form the insert.

It is envisioned in any method of forming an insert to form a support structure between at least part of the struts, which support structure is removed after forming the insert, for example by forming the support structure from a dissolvable material, for example poly(vinyl alcohol), and dissolving the support structure leaving behind the struts forming the insert.

Preferably, but not necessarily, the insert or at least part thereof is formed from a flexible material, for example thermoplastic polyurethane (TPU) or any other flexible material.

Any insert may as an option comprise one or more strips on an outer perimeter of the insert, which one or more strips face the flexible cover with an outward facing surface, wherein the outward facing surface of the one or more strips are preferably connected to the flexible cover or at least contact the flexible cover in use. A strip provides an increased surface area compared to struts spanning the same length as the strip, which increase surface area may increase adherence to the flexible cover. A strip is generally defined as a body with a length and width larger than its thickness, in particular at least 2 times, at least 5 times, or even at least 10 times larger. Typically, the length of a strip is larger than the width, in particular at least 2 times, at least 5 times, at least 10 times, or even at least 20 times larger. A strip is typically directly connected to a plurality of struts, for example 5 or more, 10 or more, or even 15 or more. Any strip may for example be 2-10 mm wide, in particular between 4-5 mm wide, and/or any strip may for example have a length between 4 and 600 mm, in particular between 300 mm and 450 mm.

Any insert disclosed herein may have a length between 200 mm and 600 mm, in particular between 300 mm and 450 mm, and/or a width between 100 mm and 400 mm, in particular between 200 mm and 300 mm, and/or a thickness between 10 and 100 mm, in particular between 10 mm and 50 mm, in any combination thereof.

In use of the water bolus, it is typically preferred to provide a flow of liquid, such as water, through the water bolus, in particular in the flexible cover and through the insert. When the insert comprises at least one closed wall element for guiding a flow of liquid through the insert, the flow of liquid through the water bolus may be controlled. The at least one closed wall prevent liquid from passing through the wall, and thus the flow of liquid is directed around the closed wall. The struts of the insert are positioned at a distance from adjacent struts, and as such liquid can typically flow between adjacent struts - provided that there is no closed wall positioned between the struts. A closed wall element is preferably fully closed - *i.e.* without any through-hole. However, in any closed wall element, one or more through-holes may be present as long as some liquid flow can also be guided around the closed wall element - next to liquid passing through any through-hole.

To further guide liquid flowing through the water bolus in use, any insert disclosed herein may comprise multiple closed wall elements for guiding a flow of liquid through the insert, for example two, three, four or even more closed wall elements. By designing and positioning the closed wall elements, the flow of liquid through the insert can be controlled.

Any closed wall element may extend from a first side of the insert to a second side of the insert opposite to the first side. The first side may correspond to a patient-facing side - also referred to as body-facing side - and the second side may face away from the patient, in use of the water bolus. When a closed wall element extends between the two sides of the insert, it may be prevented that liquid flows between the closed wall element and the inside of the flexible cover.

To reduce or even eliminate liquid flow between any closed wall element and the inside of the flexible cover, for any closed wall element, the closed wall element may extend between outer struts on the outer perimeter of the insert. Outer struts are defined as those struts which form the outer perimeter of the insert, and which may in use contact the inside of the flexible cover. In particular, any closed wall element may extend between outer struts of two sides of the insert.

For any closed wall element, the closed wall element may extend between outer struts at three sides of the insert. Two of the three sides are generally parallel - for example a body-facing side in use facing towards the patient and a side in use facing away from the towards the patient - and a third side approximately perpendicular to the other two sides.

As a particular option for any insert comprising at least two closed wall elements, a first closed wall element of the multiple closed wall elements extends over a first length, a second closed wall element of the multiple closed wall elements extends over a second length, and the first and second length at least partially do not overlap. As such, a zig-zag flow path for liquid through the insert may be achieved.

More in general, it is preferred to position at least one closed wall element of the insert such that a zig-zag flow path for liquid through the insert can be achieved. A zig-zag flow path comprises at least two distinct sections in which the flow directions for liquid flowing over the flow path are essentially in opposite directions for the respective distinct sections. It is thus envisioned that, for any insert comprising at least one closed wall element, the at least one closed wall element allows for a flow path for liquid through the insert, which flow path has a first flow path section and a second flow path section, which first flow path section and second flow path section are oriented in essentially opposite directions.

Any closed wall element disclosed herein may at least in part be substituted by a set of struts positioned together to guide water flowing through the insert in a particular direction. The struts in the set of struts are in particular positioned closer to each other than to adjacent struts which do not form part of the set of struts substituting at least part of the closed wall element. The struts in the set of struts only contact each other at outer ends of the struts, and as such a liquid flow between the struts may still be possible.

In general, struts being positioned closer to each other results in a locally increased fill factor of struts in the insert, and it is thus envisioned that a fill factor of struts in a fifth region of the insert is higher than a fill factor of struts in a sixth region of the insert, which fifth region is located adjacent to the sixth region. The fill factor may be determined by dividing the volume occupied by struts by the total volume of a particular region of the insert in which the struts are present.

A second aspect of the present disclosure provides an insert for a water bolus, wherein the insert is formed by interconnected struts which form an open structure through which a liquid can flow. The insert may be provided with any one, more or all optional features disclosed herein for any insert, in any combination.

### BRIEF DESCRIPTION OF THE FIGURES

In the figures,
FIG 1 shows an embodiment of an insert;
FIG 2A shows another embodiment of an insert;
FIG 2B schematically shows a side view of the insert of FIG 2B;
FIG 2C schematically shows a body-facing side of the insert of FIG 2B;
FIG 3A shows a mock head with inserts on opposite sides;
FIG 3B shows a water bolus;
FIG 4 shows yet another embodiment of an insert;
FIG 5A shows an insert according to the prior art; and
FIG 5B shows an insert according to the present disclosure.

It will be appreciated that the figures represent only a small number of embodiments contemplated by the present disclosure. Any insert depicted in the figures may be provided with one or more additional optional features as disclosed herein, and/or with one or more less optional features as disclosed herein.

### DETAILED DESCRIPTION OF THE FIGURES

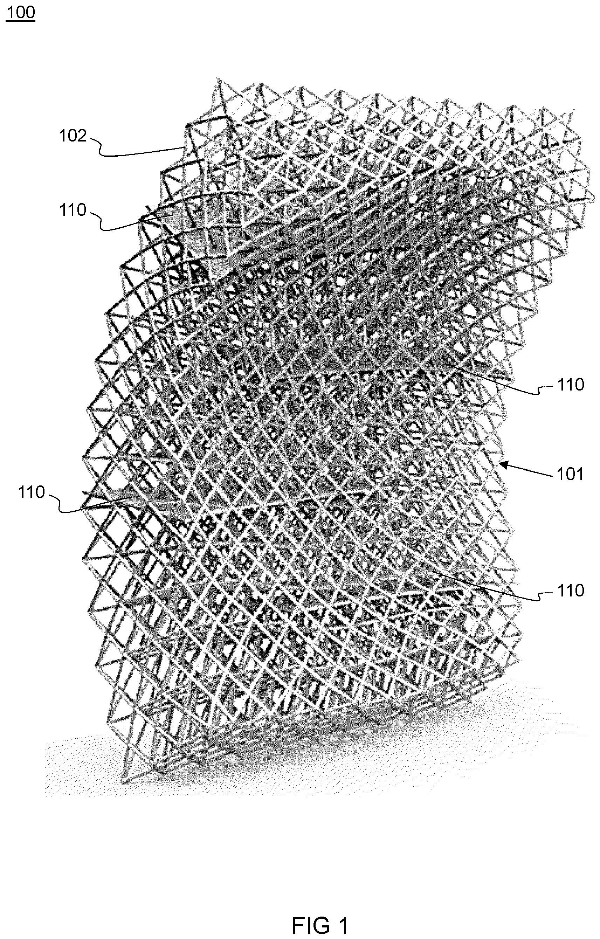

FIG 1 shows an embodiment of an insert 100 which may generally be comprised by any water bolus disclosed herein. The insert 100 comprises a plurality of interconnected struts 102 which form an open structure through which a liquid can flow. It will be appreciated that only one strut 102 is provided with a reference numeral in each of the figures, for clarity of the figures.

Struts 102 of the insert 100 are connected at their outer ends to further struts 102 of the insert 100. For any insert of the present disclosure, any number of interconnected struts can form a unit cell. The outer ends of the struts 102 form a lattice - *i.e.* a pattern of points in space. The points are connected by the struts 102. The lattice comprises points to form any number of unit cells, wherein each unit cell may comprise any number of struts 102, in any configuration. The lattice may be computer-generated based on geometry of a body part of a particular human or animal, or based on geometry of a body part of an average human or animal.

As an option depicted in FIG 1, the insert 100 comprises a plurality of closed wall elements 110. In use, the closed wall elements 110 are used for guiding a flow of liquid through the insert.

In the view of FIG 1, a body-facing side 101 of the insert 100 is shown. The body-facing side may be shaped complementary to any body part of a human or animal. Typically, for example when the body-facing side of the insert 100 is complementary to at least part of a face and/or neck of a human, the body-facing side of the insert 100 is generally concave - *i.e.* curved inwards.

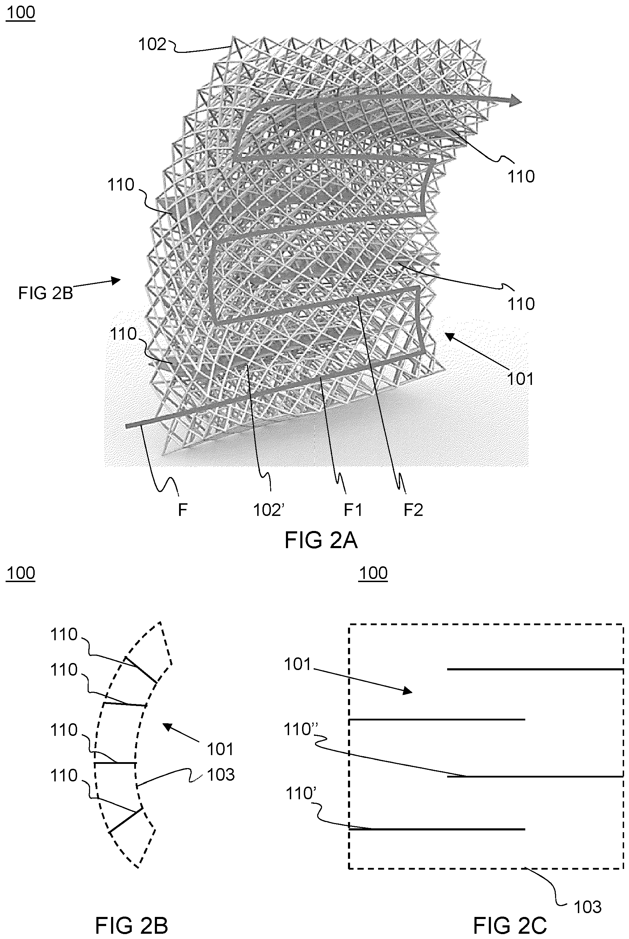

Now referring to FIG 2A, another insert 100 is depicted. The inserts of FIG 1 and 2A may be generally similar in structure, albeit differently shaped.

In FIG 2A, a flow of liquid, preferably water, is indicated with thick arrow F. The liquid flows over a flow path, of which a first flow path section F1 and second flow path section F2 are indicated in FIG 2. By virtue of the closed wall element 110, in the first flow path section F1, the liquid flows in one direction, and in the second flow path in a generally opposite direction. The liquid in use can flow around the closed wall element 110, as schematically depicted in FIG 2A. When an insert comprises multiple closed wall elements 110, any number of further flow path sections can be formed in which the direction of the liquid flow changes with respect to a further flow path section upstream.

Although in FIG 2A the flow of liquid F enters the insert 100 at a first side (left-hand), and exits the insert 100 at an opposite side (right-hand), it will be appreciated that for any water bolus, the flow of liquid may enter and exit the insert 100 also on the same side.

FIG 2B schematically shows a side view of the insert 100 of FIG 2A, showing the closed wall elements 110 and as a dashed line an outer perimeter 103 of the insert 100. Individual struts 102 are omitted from FIG 2B and FIG 2C for reasons of clarity. When a water bolus is formed comprising a flexible cover and the insert 100 of FIG 2A - or any other insert of the present disclosure - the flexible cover can be attached to the insert 100 preferably at the outer perimeter 103 of the insert 100. As such, the flexible cover can follow the shape of the outer perimeter 103 of the insert 100, in particular at at least part of the body-facing side 101 - also when the flexible cover is filled with liquid, which liquid may have a weight forcing the flexible cover away from the insert.

Whenever an insert comprises one or more closed wall elements 110, any closed wall element 110 may extend between outer struts on the outer perimeter 103 of the insert 100. For example, any closed wall element 110 may extend between an outer strut 102 on the body-facing side 101 and an outer strut 102 on the side of the insert 100 facing away from the body-facing side 101.

As a further option for any closed wall element 110, the closed wall element 110 can extend between outer struts 102 on three sides of the outer perimeter 103 of the insert 100.

FIG 2C schematically shows the body-facing 101 of the insert 100 depicted in FIG 2A. Individual struts 102 are omitted from FIG 2C for reasons of clarity. Instead, the perimeter 103 of the insert 100 is indicated with the dashed line. As a preferred option shown in FIG 2C, a first closed wall element 110' of the multiple closed wall elements extends over a first length, a second closed wall element 110" of the multiple closed wall elements extends over a second length, and the first and second length at least partially do not overlap.

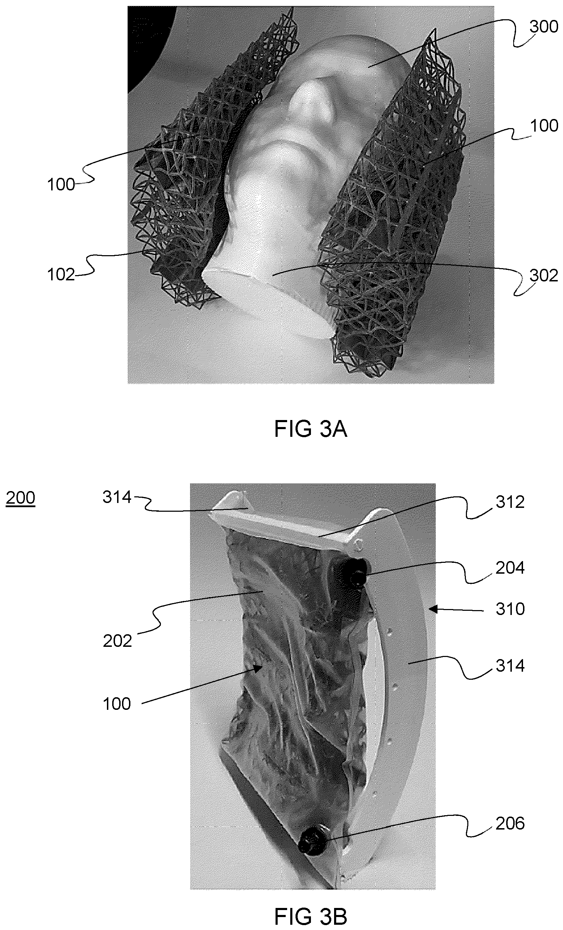

FIG 3A shows two inserts 100 according to the present disclosure, on opposite sides of a mock human head 300 and neck 302. Any insert 100 of the present disclosure may have a head-facing side - also referred to as body-facing side 101 - which is formed according to an outer shape of part of the head 300 and/or neck. Any insert 100 may be tailored to a particular head, or an insert 100 may be designed to follow an outer shape of part of an average human head, for example an average adult or child.

FIG 3B shows an example of a water bolus 200 according to the present disclosure, comprising a flexible cover 202 with an insert 100 positioned inside the flexible cover 202. The flexible cover 202 forms a container arranged to hold a volume of liquid, in particular water.

Two optional liquid ports 204, 206 through the flexible cover 202 allow a liquid to enter and respectively exit the flexible cover 202. Preferably, in use, the liquid flows through the insert 100 and allows for a homogenous temperature to form in the water bolus 200.

As a particular option depicted in FIG 3B, a support frame 310 is used to support the water bolus 200. In particular, the support frame 310 comprises one or more, preferably two, rods 312 spanning between two rigid support elements 314. The rigid support elements 314 are preferably shaped as curved elements, which in use are curved away from the water bolus 200. The flexible cover 202 can be connected to the one or more rods 312. The example of FIG 3B comprises two rods on opposite sides of the flexible cover 202, of which only one rod 312 is visible in the view of FIG 3B. The water bolus 200 is depicted without water.

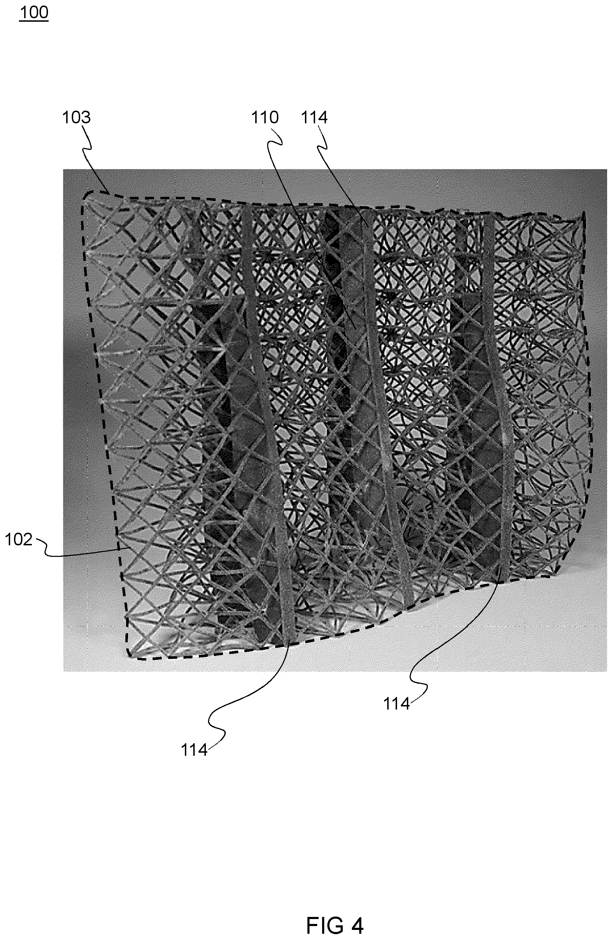

FIG 4 shows yet another example of an insert 100 according to the present disclosure. As an option applicable to also any other insert disclosed herein, the insert 100 of FIG 4 comprises one or more strips 114 on an outer perimeter of the insert. In use of the insert as part of a water bolus, the one or more strips 114 face the flexible cover with an outward facing surface. The outward facing surface of the one or more strips is preferably connected to the flexible cover, for example using an adhesive, at the body-facing side 101.

When an insert comprises at least one strip 114 and at least one closed wall element 110, the at least one strip 114 and at least one closed wall element 100 may be connected to each other directly. Preferably, the at least one strip 114 and at least one closed wall element 100 touch.

Any strip 114 may extend between the outer perimeter 103 (generally indicated with a dashed line) of the insert 100, for example between two opposite sides of the insert 100. Alternatively, any strip 114 may have one end at or near the outer perimeter 103, and the other end at a distance from the outer perimeter 103 - *i.e.* not at or near the outer perimeter 103.

As can be seen when comparing the inserts 100 depicted in FIG 2A and FIG 4, in use of the insert, any closed wall element 100 and/or strip 114 may extend generally vertically or horizontally. Alternatively, it is even envisioned that, in use of the insert, any closed wall element 100 and/or strip 114 extends at angle relative to horizontal.

Although in the figures, the closed wall elements 100 and strips 114 are depicted as being essentially straight, any closed wall element and strip may comprise any number of curved sections.

### EXAMPLE 1

An insert according to the present disclosure, and an insert according to EP3669937A1 formed by a plurality of linked, thin-walled, elongated, parallel, hollow structures, were compressed to test their stiffness (expressed in Newton/mm) - which stiffness can also be referred to as a flexibility. The inserts were positioned in a compression tester, and measurements were taken three times at slightly different positions on the respective insert, at a speed of 20 mm/min. The averages of the measurements are depicted in the table below.

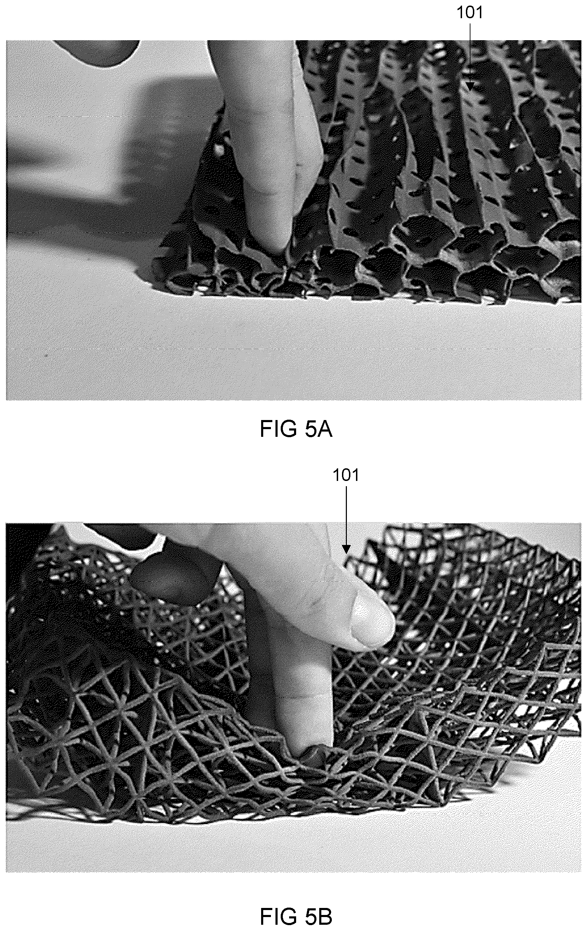

The two compared inserts essentially occupy the same volume. To compress the inserts, a pressing member with a diameter of 19 mm was pressed onto the body-facing side of the insert, and the pressing member was moved into the insert in a direction essentially normal to the body-facing side. This pressing direction is generally indicated for the insert of EP3669937A1 in FIG 5A, and for the insert of the present disclosure in FIG 5B.

| Compression | Insert according to EP3669937A1 | Insert according to the present disclosure, between closed wall elements | Insert according to the present disclosure, at closed wall elements |
|---|---|---|---|
| 5 mm | 3.1 N | 0.5 N | 2.9 N |
| 10 mm | 6.2 N | 0.7 N | 5.6 N |
| 15 mm | 11.8 N | 0.8 N | 9.4 N |
| 20 mm | 19.1 N | 0.9 N | 14.2 N |

Compared to the insert of EP3669937A1, the insert according to the present disclosure allows for increased flexibility - *i.e.* a lower stiffness - while the volume occupied by the insert remains essentially the same. The increased flexibility may allow for increased patient comfort, in particular when a water bolus is pressed against part of the face of the patient.

The present disclosure may be summarised in a non-limitative manner by way of the following numbered embodiments:
1. Water bolus (200) for covering part of an outer body surface of a body part, the water bolus comprising:
   - a flexible cover (202) for forming at least part of a container for containing a liquid; and
   - an insert (100) provided inside the flexible cover,
   wherein the insert is formed by interconnected struts (102) which form an open structure through which a liquid can flow.
2. Water bolus according to embodiment 1, wherein a majority of the struts are at both ends connected to at least two further struts.
3. Water bolus according to any of the preceding embodiments, wherein at least three struts of the insert of which outer ends are connected form a triangle.
4. Water bolus according to any of the preceding embodiments, wherein at least four struts of the insert of which outer ends are connected form a quadrilateral.
5. Water bolus according to any of the preceding embodiments, wherein at least four struts are connected at their respective outer ends and/or wherein at least five struts are connected at their respective outer ends.
6. Water bolus according to any of the preceding embodiments, wherein the insert is connected to an inside of the flexible cover, for example by an adhesive.
7. Water bolus according to any of the preceding embodiments, wherein a plurality of struts on an outer perimeter of the insert align on a smooth plane.
8. Water bolus according to any of the preceding embodiments, wherein the insert comprises one or more strips (114) on an outer perimeter of the insert, which one or more strips face the flexible cover with an outward facing surface, wherein the outward facing surface of the one or more strips are preferably connected to the flexible cover.
9. Water bolus according to any of the preceding embodiments, wherein the insert comprises at least one closed wall element (110) for guiding a flow of liquid through the insert.
10. Water bolus according to embodiment 9, wherein the insert comprises multiple closed wall elements (110) for guiding a flow of liquid through the insert.
11. Water bolus according to embodiment 10, wherein a first closed wall element of the multiple closed wall elements extends over a first length, a second closed wall element of the multiple closed wall elements extends over a second length, and the first and second length at least partially do not overlap.
12. Water bolus according to any of the embodiments 9-11, wherein the at least one closed wall element allows for a flow path (F) for liquid through the insert, which flow path has a first flow path section (F1) and a second flow path section (F2), which first flow path section and second flow path section are oriented in essentially opposite directions.
13. Water bolus according to any of the embodiments 9-12, wherein at least one closed wall element extends between outer struts on an outer perimeter of the insert.
14. Water bolus according to any of the preceding embodiments, wherein the insert is formed by a single monolithic body.
15. Water bolus according to any of the preceding embodiments, wherein the container formed by the flexible cover holds a volume of water, which volume of water is at least partially positioned between struts of the insert.
16. Water bolus according to any of the preceding embodiments, wherein the insert has a stiffness against compression between 0.1 and 15 N/mm.
17. Water bolus according to any of the preceding embodiments, wherein a length of one or more struts in a first region of the insert is shorter than a length of one or more struts in a second region of the insert, which second region is located adjacent to the first region.
18. Water bolus according to any of the preceding embodiments, wherein a cross-sectional area of one or more struts in a third region of the insert is different from a cross-sectional area of one or more struts in a fourth region of the insert, which fourth region is located adjacent to the third region.
19. Water bolus according to any of the preceding embodiments, wherein a fill factor of struts in a fifth region of the insert is higher than a fill factor of struts in a sixth region of the insert, which fifth region is located adjacent to the sixth region.
20. Insert for a water bolus according to any of the preceding embodiments, wherein the insert is formed by interconnected struts which form an open structure through which a liquid can flow.

## Claims

1. Water bolus (200) for covering part of an outer body surface of a body part, the water bolus comprising:
- a flexible cover (202) for forming at least part of a container for containing a liquid; and
- an insert (100) provided inside the flexible cover,
wherein the insert is formed by interconnected struts (102) which form an open structure through which a liquid can flow.

2. Water bolus according to claim 1, wherein a majority of the struts are at both ends connected to at least two further struts.

3. Water bolus according to any of the preceding claims, wherein at least three struts of the insert of which outer ends are connected form a triangle, and/or wherein at least four struts of the insert of which outer ends are connected form a quadrilateral, and/or wherein at least four struts are connected at their respective outer ends and/or wherein at least five struts are connected at their respective outer ends.

4. Water bolus according to any of the preceding claims, wherein the insert is connected to an inside of the flexible cover, for example by an adhesive, preferably wherein a plurality of struts on an outer perimeter of the insert align on a smooth plane.

5. Water bolus according to any of the preceding claims, wherein the insert comprises one or more strips (114) on an outer perimeter of the insert, which one or more strips face the flexible cover with an outward facing surface, wherein the outward facing surface of the one or more strips are preferably connected to the flexible cover.

6. Water bolus according to any of the preceding claims, wherein the insert comprises at least one closed wall element (110) for guiding a flow of liquid through the insert.

7. Water bolus according to claim 6, wherein the insert comprises multiple closed wall elements (110) for guiding a flow of liquid through the insert, in particular wherein a first closed wall element of the multiple closed wall elements extends over a first length, a second closed wall element of the multiple closed wall elements extends over a second length, and the first and second length at least partially do not overlap.

8. Water bolus according to any of the claims 6-7, wherein the at least one closed wall element allows for a flow path (F) for liquid through the insert, which flow path has a first flow path section (F1) and a second flow path section (F2), which first flow path section and second flow path section are oriented in essentially opposite directions.

9. Water bolus according to any of the claims 6-8, wherein at least one closed wall element extends between outer struts on an outer perimeter of the insert.

10. Water bolus according to any of the preceding claims, wherein the insert is formed by a single monolithic body.

11. Water bolus according to any of the preceding claims, wherein the container formed by the flexible cover holds a volume of water, which volume of water is at least partially positioned between struts of the insert.

12. Water bolus according to any of the preceding claims, wherein the insert has a stiffness against compression between 0.1 and 15 N/mm.

13. Water bolus according to any of the preceding claims, wherein a length of one or more struts in a first region of the insert is shorter than a length of one or more struts in a second region of the insert, which second region is located adjacent to the first region and/or wherein a cross-sectional area of one or more struts in a third region of the insert is different from a cross-sectional area of one or more struts in a fourth region of the insert, which fourth region is located adjacent to the third region.

14. Water bolus according to any of the preceding claims, wherein a fill factor of struts in a fifth region of the insert is higher than a fill factor of struts in a sixth region of the insert, which fifth region is located adjacent to the sixth region.

15. Insert for a water bolus according to any of the preceding claims, wherein the insert is formed by interconnected struts which form an open structure through which a liquid can flow.
